# EUROPÄISCHE PATENTSCHRIFT

(11) **EP 4 125 719 B1**
(45) Veröffentlichungstag und Bekanntmachung des Hinweises auf die Patenterteilung: **23.09.2026**
(21) Anmeldenummer: 21716137.1
(22) Anmeldetag: 29.03.2021
(51) Int. Cl.: A61F 2/16

(54) **INTRAOKULARLINSEN MIT INTEGRIERTER ARRETIEREINHEIT FÜR EINEN HAPTIKBÜGEL**
INTRAOCULAR LENSES WITH INTEGRATED LOCKING UNIT FOR A HAPTIC CLAMP
LENTILLES INTRAOCULAIRES À UNITÉ DE VERROUILLAGE INTÉGRÉE POUR UNE PINCE HAPTIQUE

(30) Priorität: 07.04.2020 DE 102020109639
(43) Veröffentlichungstag der Anmeldung: 08.02.2023
(73) Patentinhaber: Carl Zeiss Meditec AG, 07745 Jena (DE)
(72) Erfinder: SRBINOSKA, Hristina, 14532 Kleinmachnow (DE)
(74) Vertreter: Hofstetter, Schurack & Partner
(86) Internationale Anmeldenummer: PCT/EP2021/058127
(87) Internationale Veröffentlichungsnummer: WO 2021/204576

(56) Entgegenhaltungen:
- EP-A1- 0 175 972
- US-A- 4 280 232

## Beschreibung

### Technisches Gebiet

Ein Aspekt der Erfindung betrifft eine Intraokularlinse mit zumindest einem optischen Teil und einer Haptik, die mit dem optischen Teil gekoppelt ist. Die Intraokularlinse weist eine optische Hauptachse auf, die eine Vorderseite und eine Rückseite des optischen Teils durchdringt. Die Haptik weist zumindest einen ersten Haptikbügel auf.

### Stand der Technik

Intraokularlinsen sind in vielfältigen Ausgestaltungen bekannt. Sie weisen einen optischen Teil auf. An diesen grenzt an einem radial äußeren Rand eine Haptik an. Die Haptik kann sehr vielfältig ausgebildet sein. Bekannt sind Haptiken, bei denen zumindest ein Haptikbügel an dem optischen Teil abstehend angeordnet ist. Der Haptikbügel kann strangartig ausgebildet sein. Mit seinen beiden Enden kann er an den optischen Teil münden. Darüber hinaus sind auch Ausführungen eines Haptikbügels bekannt, der nur mit einem Ende an dem optischen Teil mündet. Das andere Ende ist frei kragend. Diesbezüglich werden solche Haptikbügel auch als C-Bügel bezeichnet.

Intraokularlinsen, die in einen Kapselsack eines Auges implantiert werden, können auch mit dem Kapselsack verwachsen. Dabei verwächst die Haptik mit dem Kapselsack. Die Intraokularlinse ist dann ziemlich positionsfixiert und unverrückbar am Kapselsack angeordnet. Muss die Linse in einem derartigen Zustand im Kapselsack in ihrer Position geändert werden, so ist dies schwierig. Die mit dem Kapselsack verwachsene Haptik muss in dem Zusammenhang erst gelöst werden, um eine gewünschte Drehung durchführen zu können. Gerade bei Intraokularlinsen, die eine spezifische Abbildungseigenschaft aufweisen, beispielsweise eine torische Intraokularlinse, kann sich eine derartige Positionsjustierung im Kapselsack ergeben. Dies dahingehend, um dann den Astigmatismus, der mit der torischen Intraokularlinse korrigiert werden soll, bestmöglich korrigieren zu können.

Aus der US 4504981 ist eine Intraokularlinse mit einem optischen Teil und einer Haptik, die zwei Haptikbügel aufweist, bekannt. An einem Haptikbügel ist ein Loch ausgebildet. Darüber hinaus ist in dem optischen Teil ein Loch ausgebildet. Mit einem zur Intraokularlinse separaten Hilfswerkzeug kann in das Loch in dem Haptikbügel eingegriffen werden. Der Haptikbügel kann zu dem optischen Teil hingezogen werden. Wird das Hilfswerkzeug dann auch noch zusätzlich in das Loch in dem optischen Teil eingeführt, so kann die zum optischen Teil mit dem Hilfswerkzeug herangezogene Position des Haptikbügels fixiert werden. Es ist dort jedoch erforderlich, dass ein Hilfswerkzeug, welches nicht Bestandteil der Intraokularlinse ist, als separate Haltekomponente genutzt wird, um überhaupt den Haltebügel an dem optischen Teil fixieren zu können. Eine derartige Ausgestaltung erfordert stets ein derartiges separates Hilfs- und Haltewerkzeug.

Darüber hinaus ist in dem Stand der Technik die Positionsfixierung des Haltebügels zum optischen Teil lediglich für den eigentlichen Implantationsvorgang der Intraokularlinse vorgesehen. Es soll nämlich damit vermieden werden, dass der relativ filigrane und frei kragende Haltebügel unerwünscht komprimiert oder in seiner Orientierung unerwünscht verändert werden würde. Die dortige Lösung ist daher grundsätzlich nicht tauglich, um eine bereits implantierte Intraokularlinse, die mit einem Kapselsack verwachsen ist, schonend von dem Kapselsack zu lösen, um dann im implantierten Zustand eine Rotation der Intraokularlinse in dem Kapselsack bewirken zu können.

Aus der US 4 280 232 A und der EP 0 175 972 A1 sind Intraokularlinsen mit spezifischen Haptiken bekannt.

### Darstellung der Erfindung

Es ist Aufgabe der vorliegenden Erfindung, eine Intraokularlinse zu schaffen, die eine verbesserte fixierende Positionierung von Komponenten der Intraokularlinse zueinander ermöglicht.

Diese Aufgabe wird durch eine künstliche Intraokularlinse gemäß den Merkmalen des Anspruchs 1 gelöst.

Ein Aspekt der Erfindung betrifft eine Intraokularlinse. Die Intraokularlinse weist zumindest einen optischen Teil auf. Darüber hinaus weist die Intraokularlinse eine Haptik auf. Die Haptik ist mit dem optischen Teil gekoppelt. Insbesondere ist sie direkt mit dem optischen Teil verbunden. Die Intraokularlinse weist eine optische Hauptachse auf, die eine Vorderseite und eine Rückseite des optischen Teils durchdringt, insbesondere zentral mittig durchdringt. Die Haptik weist zumindest einen ersten Haptikbügel auf. An dem Haptikbügel ist ein Koppelelement der Intraokularlinse integriert. Das Koppelelement ist somit einstückig mit dem Haptikbügel ausgebildet. An dem optischen Teil oder an einem mit dem optischen Teil gekoppelten Anschluss ist ein Gegenkoppelelement der Intraokularlinse integriert. Ein Gegenkoppelelement kann somit auch an einem den optischen Teil vollständig oder teilweise umgebenden Anschluss ausgebildet sein. Das Gegenkoppelelement ist einstückig mit dem optischen Teil oder dem Anschluss ausgebildet. Der Anschluss weist keine optische Abbildungseigenschaft auf. Er schließt direkt an den Umfangsrand des optischen Teils an. Der Anschluss in Form eines Segmentstückes kann ein Ausschnitt eines um den optischen Teil umlaufenden Rings sein. Der Anschluss kann aber auch ein geometrisch beliebig ausgebildeter Anschluss an den optischen Teil sein, wobei der Anschluss keine Ringgeometrie oder einen Ausschnitt eines Ringes bildet. Der Anschluss kann eine zur Haptik separate Komponente der Intraokularlinse sein. Der Anschluss kann vollständig umlaufend um den optischen Teil ausgebildet sein, beispielsweise als Ring, oder nur teilweise umlaufend um den optischen Teil ausgebildet sein. Der Anschluss ist insbesondere am Äquator des optischen Teils angeordnet. Der Anschluss ist daher insbesondere bevorzugt um die optische Hauptachse zumindest bereichsweise umlaufend angeordnet. Der Anschluss ist insbesondere einstückig mit dem optischen Teil ausgebildet.

Das Koppelelement ist zum direkten Koppeln mit dem Gegenkoppelelement ausgebildet. Im gekoppelten Zustand von Koppelelement und Gegenkoppelelement ist ein Außenrandsegment des Haptikbügels in einem radial geringeren Abstand zum optischen Teil angeordnet als im ungekoppelten Zustand von Koppelelement und Gegenkoppelelement. Durch das Koppelelement und das Gegenkoppelelement ist somit eine in die Intraokularlinse integrierte Arretiereinheit geschaffen. Damit ist es ermöglicht, dass ein Haptikbügel in der definierten Position zum optischen Teil gehalten ist. Diese beiden integrierten Elemente sind im gekoppelten Zustand direkt miteinander gekoppelt. Dadurch kann durch diese Elemente selbst der gekoppelte Zustand dauerhaft gehalten werden. Es ist somit ermöglicht, dass keine separaten Halteteile erforderlich sind, um einen derartig definierten gekoppelten Zustand, bei welchem der Haptikbügel in einer radial nach innen gezogenen Position zum optischen Teil fixiert ist, zu halten. Durch eine derartige Ausgestaltung ist es auch erreicht, dass durch das direkte Koppeln des Koppelelements und des Gegenkoppelelements die Beschädigung der Intraokularlinse, insbesondere im optischen Teil, vermieden wird. Eine derartige Gefahr kann dann auftreten, wenn ein zur Intraokularlinse externes Halteteil zum Halten einer derartigen Koppelposition zwischen dem Haltebügel und dem optischen Teil erforderlich ist. Da ein Außenrandsegment des Haptikbügels im gekoppelten Zustand radial zum ersten optischen Teil gezogen ist und somit radial vorgespannt ist, wirken auf das Koppelelement und das Gegenkoppelelement entsprechende Kräfte. Diese können durch diese gekoppelten Elemente jedoch gehalten werden. Aufgrund der Ausgestaltung des Koppelelements und des Gegenkoppelelements können Beschädigungen dieser Elemente einerseits und des optischen Teils andererseits vermieden werden.

Im ungekoppelten Zustand zwischen dem Koppelelement und dem Gegenkoppelelement ist der Haptikbügel in einer Grundposition zum optischen Teil angeordnet. In dieser Grundposition ist ein Außenrandsegment des Haptikbügels radial weiter entfernt vom optischen Teil als im gekoppelten Zustand von dem Koppelelement mit dem Gegenkoppelelement.

Durch eine derartige Ausgestaltung einer Intraokularlinse selbst ist es in sehr vorteilhafter Weise ermöglicht, eine mit dem Kapselsack verwachsene Haptik einfacher lösen zu können. Es ist in dem Zusammenhang dann nur noch erforderlich, mit einem Hilfswerkzeug das Koppelelement zu dem optischen Teil hin zu ziehen. Insbesondere ist dadurch ein Lösen des verwachsenen Haptikbügels vom Kapselsack bewirkt. Indem dann mit diesem Hilfswerkzeug das Koppelelement so in Position gebracht wird, dass es mit dem Gegenkoppelelement koppelt, kann dieser gekoppelte Zustand ohne weitere Betätigung und ohne weiteres Vorhandensein des Hilfswerkzeugs gehalten werden. Es ist also das Hilfswerkzeug nur für die Handlung erforderlich, um das Koppelelement zum Gegenkoppelelement hin zu bringen. Der dann erreichte gekoppelte Zustand wird dann ausschließlich durch das Koppelelement und das Gegenkoppelelement der Intraokularlinse selbst aufrechterhalten.

Ein Haptikbügel weist insbesondere zumindest ein Ende auf, das mit dem optischen Teil oder dem Anschluss an einer ersten Stelle direkt verbunden ist, insbesondere ohne Zerstörung unlösbar damit verbunden ist. Das Koppelelement ist an dem Haptikbügel entfernt zu diesem Ende an dem Haptikbügel angeordnet, insbesondere einstückig damit hergestellt. Das Ende ist unabhängig vom gekoppelten Zustand des Koppelelements mit dem Gegenkoppelelement als auch unabhängig vom entkoppelten Zustand des Koppelelements mit dem Gegenkoppelelement dauerhaft mit dem optischen Teil oder dem Anschluss verbunden, insbesondere ohne örtliche Änderung des Endes relativ zum optischen Teil oder zum Anschluss. Es kann vorgesehen sein, dass ein Haptikbügel nur mit einem Ende an den Anschluss oder den optischen teil endet und das andere Ende des Haptikbügels frei kragend angeordnet ist. Weist ein Haptikbügel in einem Ausführungsbeispiel ein weiteres Ende auf, das an einer zur ersten unterschiedlichen zweiten Stelle mit dem optischen Teil oder dem Anschluss verbunden ist, gelten die entsprechenden Erläuterungen in Bezug zum Koppelelement, wie sie oben für das erste Ende dargelegt sind. Beide Enden enden dann an dem Anschluss oder dem optischen Teil, jedoch insbesondere an unterschiedlichen Stellen. Die Koppelstelle zwischen dem Koppelelement und dem Gegenkoppelelement ist entfernt und unabhängig von der ersten Stelle oder der zweiten Stelle am optischen Teil oder am Anschluss ausgebildet. Der optische Teil ist einstückig mit zumindest einem Haptikbügel, insbesondere allen Haptikbügel der Linse hergestellt. Das bedeutet, dass beim grundsätzlichen Herstellungsprozess der optische Teil, gegebenenfalls mit dem Anschluss, zusammen mit dem Haptikbügel gefertigt wird.

Ein Koppelelement ist ein zu einem Ende eines Haptikbügels unterschiedliches Bauteil eines Haptikbügels. Ein Koppelelement ist beabstandet zu einem Ende eines Haptikbügels an dem Haptikbügel angeordnet.

In einem Ausführungsbeispiel ist die Intraokularlinse mit nur einem einzigen, einstückigen optischen Teil ausgebildet. Dieser optische Teil ist insbesondere einstückig mit der Haptik ausgebildet. Die Intraokularlinse ist dann als Ganzes einstückig ausgebildet.

In einem anderen Ausführungsbeispiel ist die Intraokularlinse mit einem ersten, einstückigen optischen Teil ausgebildet. Dieser erste optische Teil kann einstückig mit der Haptik ausgebildet sein. Die Intraokularlinse weist bei diesem Ausführungsbeispiel einen zweiten optischen Teil auf. Dieser zweite optische Teil ist separat zum ersten optischen Teil. Der zweite optische Teil bildet mit dem ersten optischen Teil ein Optiksystem der Intraokularlinse. Es kann bei diesem Ausführungsbeispiel vorgesehen sein, dass die beiden optischen Teile aneinander anliegen. Sie können in Richtung der optischen Hauptachse in Reihe zueinander angeordnet sein. Die zumindest zwei optischen Teile können durch ein Verbindungselement, beispielsweise einen Verbindungsbügel, der Intraokularlinse miteinander verbunden sein. Es kann bei dem Ausführungsbeispiel vorgesehen sein, dass das zumindest eine Gegenkoppelelement in dem zweiten optischen Teil ausgebildet ist. Insbesondere ist der zweite optische Teil in Richtung senkrecht zur optischen Hauptachse größer ausgebildet, als der erste optische Teil. Dadurch weist der zweite optische Teil einen radialen Überstand im Vergleich zum ersten optischen Teil auf. In diesem Überstand ist insbesondere das zumindest eine Gegenkoppelelement ausgebildet.

In einem weiteren Ausführungsbeispiel weist die Intraokularlinse einen optischen Teil und eine dazu separate Haptik auf. Die beiden Komponenten sind bei diesem Ausführungsbeispiel nicht einstückig ausgebildet. Die Haptik bildet insbesondere ein Trägerskelett mit Haptikbügel und einer Aufnahme für den optischen Teil. Diese Aufnahme kann ein Ring oder ein Segmentstück sein. Der optische Teil ist in die Aufnahme einsetzbar, so dass der optische Teil an seinem Äquator zumindest bereichsweise von der Aufnahme umgriffen ist und an dieser gehalten ist. Das zumindest eine Gegenkoppelelement ist in dem optischen Teil ausgebildet. Es kann aber auch in der Aufnahme ausgebildet sein.

Das Koppelelement weist ein Verbindungsteil auf, mit welchem das Koppelelement mit dem Haptikbügel direkt verbunden ist. Das Koppelelement weist darüber hinaus einen Koppelsteg auf. Dieser Koppelsteg ist an dem Verbindungsteil endseitig ausgebildet. Insbesondere ist der Koppelsteg zum direkten Eingriff in das Gegenkoppelelement vorgesehen. In einer derartigen Ausgestaltung greifen das Koppelelement und das Gegenkoppelelement ineinander ein. Es ist somit eine mechanische, ineinandergreifende Wirkverbindung als Formschluss geschaffen. Eine besonders einfache und dennoch mechanisch belastbare direkte Verbindung zwischen diesen beiden Elementen ist dadurch geschaffen.

Durch das Verbindungsteil ist eine stabile Anbindungseinheit an den Haptikbügel geschaffen. In der Schnittstelle zwischen dem Haptikbügel und dem Koppelelement ist somit eine auch belastbare Anbindungsstelle geschaffen. Ein Abreißen des Koppelelements von dem Haptikbügel ist dadurch verhindert. Da gerade an dieser Stelle auch entsprechende Zugkräfte wirken, wenn der Haptikbügel aus seinem verwachsenen Zustand mit dem Kapselsack gelöst werden soll, ist eine derartige Ausgestaltung diesbezüglich vorteilhaft.

Darüber hinaus dient somit dieses Verbindungsteil auch als robuster und belastbarer Träger für den Koppelsteg.

Das Verbindungsteil weist zumindest einen Verbindungsbalken auf. Dieser erstreckt sich von einer Innenseite des Haptikbügels abstehend in radialer Richtung zur optischen Hauptachse zum optischen Teil hin. Die Innenseite des Haptikbügels ist dabei dem optischen Teil zugewandt. Durch eine derartige Anbindungsstelle und Orientierung dieses Verbindungsbalkens können die oben genannten Vorteile nochmals unterstützt werden. Gerade durch eine geradlinige Ausgestaltung des Verbindungsbalkens können auch die Zugkräfte eines Hilfswerkzeugs von dem Verbindungsteil auf den Haptikbügel sehr vorteilhaft übertragen werden. Auch ein sehr geradliniges Ziehen und eine direkte Kraftübertragung sind dadurch ermöglicht. Der Verbindungsbalken ist in seiner geradlinigen Ausgestaltung auch einfach herzustellen. Es ist eine besonders einfache Formgebung realisiert.

Insbesondere ist der Verbindungsbalken in Richtung der optischen Hauptachse betrachtet dünner als der Haptikbügel, an dem der Verbindungsbalken endet.

In einem Ausführungsbeispiel bilden der Verbindungsbalken und der daran endseitig angeformte Koppelsteg ein im Querschnitt L-förmiges Teil. Die oben genannten Vorteile kommen in dem Zusammenhang besonders zum Tragen. Dieses Teil weist eine einfache Formgebung auf. Es ist mechanisch stabil und entsprechend belastbar. Dennoch ist durch den Bodenschenkel der L-Form ein einfacher Koppelsteg gebildet, der einerseits durch ein Hilfswerkzeug leicht umgriffen werden kann, um somit eine Zugkraft vom Hilfswerkzeug auf den Verbindungsbalken und von dort auf den Haptikbügel sehr direkt zu übertragen. Andererseits ist durch diese L-Form auch ein einfaches Koppeln mit dem Gegenkoppelelement ermöglicht. Es muss nur dieser als L-Schenkel ausgebildete Koppelsteg in das Gegenkoppelelement eingreifen, um den gekoppelten Zustand zu erreichen und auch aufrecht zu erhalten. Die L-Form bildet dabei auch eine Ausgestaltung, die sehr formstabil bleibt. Somit wird auch der gekoppelte Zustand dauerhaft stabil gehalten. Der längere L-Schenkel ist durch den Verbindungsbalken gebildet.

Das Verbindungsteil ist im ungekoppelten Zustand zwischen dem Koppelelement und dem Gegenkoppelelement frei kragend angeordnet. Das Verbindungsteil ist in diesem ungekoppelten Zustand in Richtung des optischen Teils orientiert. Das Verbindungsteil ist in diesem ungekoppelten Zustand berührungslos zum optischen Teil angeordnet. Durch diese Positionierungen und Orientierungen ergeben sich Vorteile bezüglich der Handhabung mit einem Hilfswerkzeug einerseits und der Aufrechterhaltung des gekoppelten Zustands andererseits.

In einem Ausführungsbeispiel ist der gekoppelte Zustand durch das direkte Ineinandergreifen zwischen dem Koppelelement und dem Gegenkoppelelement selbstständig und ohne weitere externe Halteteile nur durch diese beiden Elemente gehalten. Eine sehr komponentenreduzierte und dennoch mechanisch belastbare und stabile Verbindung ist dadurch ermöglicht.

Es kann vorgesehen sein, dass die gesamte Intraokularlinse einstückig ausgebildet ist. Möglich ist es auch, dass die Haptik unabhängig von dem optischen Teil hergestellt ist und der optische Teil dann in diese Haptik eingesetzt ist beziehungsweise daran gehalten ist. In dem Zusammenhang kann der optische Teil aus einem Polymermaterial ausgebildet sein. Die Haptik kann als Spritzgussbauteil oder als 3D-Druckbauteil realisiert sein. Möglich ist es jedoch auch, dass die gesamte Intraokularlinse mit dem optischen Teil und der Haptik aus einem Stück hergestellt ist, insbesondere aus einem Polymermaterial.

In einem Ausführungsbeispiel ist vorgesehen, dass das Koppelelement ein Verbindungsteil aufweist, mit welchem das Koppelelement mit dem Haptikbügel direkt verbunden ist. Das Verbindungsteil weist zumindest zwei separate, insbesondere geradlinige, Verbindungsbalken auf. Diese zumindest zwei Verbindungsbalken sind an unterschiedlichen Stellen mit einer Innenseite des Haptikbügels verbunden, insbesondere einstückig. Diese beiden Verbindungsbalken stehen in einer vorteilhaften Ausführung an unterschiedlichen Stellen von einer Innenseite des Haptikbügels ab und sind in Richtung des optischen Teils orientiert.

In einem vorteilhaften Ausführungsbeispiel sind diese Verbindungsbalken an ihren Enden, die dem optischen Teil zugewandt sind, miteinander verbunden. Insbesondere können sie an diesen Enden einstückig miteinander ausgebildet sein. Dadurch ergibt sich ein Koppelelement, welches an unterschiedlichen Stellen mit dem Haptikbügel verbunden ist. Damit kann eine höhere Zugkraft aufgebracht werden, sodass sich der Haptikbügel auch bei starker Verwachsung zuverlässig vom Kapselsack entfernen lässt. Die dann erforderliche und gewollte Rotation der Intraokularlinse relativ zum Kapselsack ist dann einfacher möglich.

In einem Ausführungsbeispiel ist das Gegenkoppelelement ein Durchgangsloch. Dies bedeutet, dass es vollständig durchgängig ausgebildet ist. Damit ist es auch erreicht, dass durch dieses Durchgangsloch eine Flüssigkeit eingebracht werden kann. Dadurch ist ein Einbringen dieser Flüssigkeit zwischen dem Kapselsack und dem optischen Teil der Intraokularlinse einfach ermöglicht.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder abweichen.

Die in den Unterlagen angegebenen konkreten Werte von Parametern und Angaben zu Verhältnissen von Parametern bzw. Parameterwerten zur Definition von Ausführungsbeispielen der Augenlinse sind auch im Rahmen von Abweichungen, beispielsweise aufgrund von Messfehlern, Systemfehlern, DIN-Toleranzen etc. als vom Rahmen der Erfindung mit umfasst anzusehen, wodurch auch Erläuterungen, die sich auf im Wesentlichen entsprechende Werte und Angaben beziehen darunter zu verstehen sind.

### Kurze Beschreibung der Zeichnungen

Ausführungsbeispiele der Erfindung werden nachfolgend anhand schematischer Zeichnungen näher erläutert. Es zeigen:
- Fig. 1a: eine perspektivische Darstellung eines ersten Ausführungsbeispiels einer erfindungsgemäßen Intraokularlinse;
- Fig. 1b: eine perspektivische Darstellung eines weiteren Ausführungsbeispiels einer erfindungsgemäßen Intraokularlinse;
- Fig. 2: eine Draufsicht auf ein Ausführungsbeispiel einer erfindungsgemäßen Intraokularlinse in einem ungekoppelten Zustand zwischen einem Koppelelement und einem Gegenkoppelelement;
- Fig. 3: eine Schnittdarstellung durch die Intraokularlinse gemäß Fig. 2;
- Fig. 4: eine Draufsicht auf die Intraokularlinse gemäß Fig. 2, jedoch in einem gekoppelten Zustand zwischen einem Koppelelement und einem Gegenkoppelelement;
- Fig. 5: eine Schnittdarstellung durch die Intraokularlinse gemäß Fig. 4;
- Fig. 6: eine weitere Schnittdarstellung entsprechend der Ansicht in Fig. 5, jedoch in einer zur Fig. 5 gezeigten Alternative eines gekoppelten Zustands zwischen einem Koppelelement und einem Gegenkoppelelement;
- Fig. 7: eine Schnittdarstellung eines nicht von der Erfindung umfassten Ausführungsbeispiels einer Intraokularlinse in einem gekoppelten Zustand zwischen einem Koppelelement und einem Gegenkoppelelement, wobei hier das Koppelelement und das Gegenkoppelelement alternativ zu den Ausführungen gemäß Fig. 2 bis Fig. 6 gestaltet sind;
- Fig. 8: eine schematische Draufsicht auf ein weiteres Ausführungsbeispiel einer Intraokularlinse im ungekoppelten Zustand eines Koppelements und eines Gegenkoppelelements;
- Fig. 9: eine Schnittdarstellung durch ein weiteres Ausführungsbeispiel einer Intraokularlinse im ungekoppelten Zustand von Koppelelementen und eines Gegenkoppelelementen; und
- Fig. 10: eine Schnittdarstellung durch ein weiteres Ausführungsbeispiel einer Intraokularlinse im ungekoppelten Zustand von Koppelelementen und eines Gegenkoppelelementen. Bevorzugte Ausführungsbeispiele der Erfindung

In den Figuren werden gleiche oder funktionsgleiche Elemente mit den gleichen Bezugszeichen versehen.

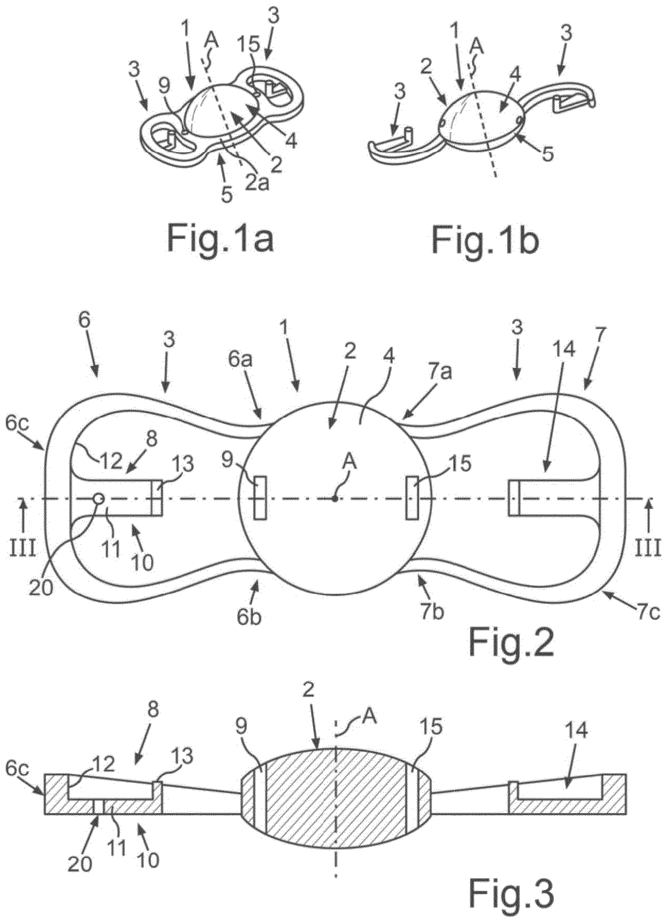

In Fig. 1a ist in einer perspektivischen Darstellung ein erstes Ausführungsbeispiel einer künstlichen Augenlinse gezeigt, die eine Intraokularlinse 1 ist. Die Intraokularlinse 1 weist einen optischen Teil 2 und daran anschließend eine Haptik 3 auf. Die Intraokularlinse 1 ist faltbar und über einen Kleinschnitt in ein Auge einführbar. Der optische Teil 2, der wesentlich für die optische Abbildungseigenschaft der Augenlinse 1 ist, weist eine optische Hauptachse A auf. Der optische Teil 2 weist darüber hinaus in Richtung dieser optischen Hauptachse A betrachtet eine erste optische Fläche bzw. Seite 4 auf, die eine Vorderseite sein kann, und weist gegenüberliegend eine zweite optische Fläche bzw. Seite 5 auf, die eine Rückseite sein kann. Die beispielhafte Vorderseite ist im implantierten Zustand der Augenlinse 1 im Auge der Hornhaut zugewandt, wohingegen die Rückseite dieser Hornhaut abgewandt ist.

In Fig. 1b ist in einer perspektivischen Darstellung ein weiteres Ausführungsbeispiel einer als Intraokularlinse 1 ausgebildeten künstlichen Augenlinse gezeigt. Sie unterscheidet sich von der Ausführung in Fig. 1a durch die unterschiedliche Haptik 3. Mittels der Haptik 3 ist die Intraokularlinse 1 im Auge gehalten.

Die Seiten 4 und 5 sind in den Ausführungen insbesondere uneben, insbesondere konvex, gekrümmt. Die Seiten 4 und/oder 5 können auch konkav oder eben sein. Auf zumindest einer Seite 4, 5 kann auf dieser konvexen Grundform ein diffraktives Profil ausgebildet sein.

In Fig. 2 ist in einer schematischen Draufsicht die Intraokularlinse 1 gemäß dem Ausführungsbeispiel in Fig. 1a gezeigt. Die Haptik 3 weist im Ausführungsbeispiel einen ersten Haptikbügel 6 und einen zweiten Haptikbügel 7 auf. Die beiden separaten Haptikbügel 6 und 7 sind an unterschiedlichen Umfangspositionen mit dem optischen Teil 2 verbunden. Der Haptikbügel 6 ist im gezeigten Ausführungsbeispiel strangartig ausgebildet. Er ist mit einem ersten Ende 6a mit dem optischen Teil 2 verbunden. Mit einem gegenüberliegenden zweiten Ende 6b ist er ebenfalls mit dem optischen Teil 2 verbunden. Entsprechendes ist im Ausführungsbeispiel mit Enden 7a und 7b des zweiten Haptikbügels 7 vorgesehen. Die Haptikbügel 6, 7 sind im Ausführungsbeispiel U-förmig.

An dem ersten Haptikbügel 6 ist ein Koppelelement 8 integriert. Dies bedeutet, dass dieses Koppelelement 8 einstückig mit dem Haptikbügel 6 ausgebildet ist. In dem optischen Teil 2 ist ein Gegenkoppelelement 9 ausgebildet. Das Gegenkoppelelement 9 kann beispielsweise ein Durchgangsloch sein. Es kann jedoch auch eine einfache Vertiefung oder Mulde sein. Insbesondere kann es auch ein Sackloch sein.

Das Koppelelement 8 ist zum direkten Koppeln mit dem Gegenkoppelelement 9 ausgebildet. Das Koppelelement 8 und das Gegenkoppelement 9 sind so ausgebildet, dass in deren gekoppelten Zustand der Haptikbügel 6 in radialer Richtung zur optischen Hauptachse A betrachtet in einer zum optischen Teil 2 näheren Position gehalten ist, als in der in Fig. 2 gezeigten ungekoppelten Position beziehungsweise im ungekoppelten Zustand. Wie zu erkennen ist, weist das Koppelelement 8 ein Verbindungsteil 10 auf. Mit diesem Verbindungsteil 10, welches hier zumindest einen Verbindungsbalken 11 aufweist, ist das Koppelelement 8 direkt mit einer Innenseite 12 des Haptikbügels 6 verbunden, insbesondere einstückig damit ausgebildet. Das Verbindungsteil 8 weist darüber hinaus einen Koppelsteg 13 auf. Der Koppelsteg 13 ist an dem Verbindungsteil 10 endseitig ausgebildet. Insbesondere ist der Koppelsteg 13 an demjenigen Ende des Verbindungsteils 10 angeordnet, welches dem optischen Teil 2 zugewandt ist. Insbesondere ist der Verbindungsbalken 11 geradlinig ausgebildet. Wie zu erkennen ist, erstreckt er sich von der Innenseite 12 zum optischen Teil 2 hin. Der Verbindungsbalken 11 ist insbesondere hohlraumfrei ausgebildet. Der Verbindungsbalken 11 steht von der Innenseite 12 ab und erstreckt sich in Richtung des optischen Teils 2.

In dem ungekoppelten Zustand, wie er in Fig. 2 gezeigt ist, ist das Koppelelement 8 beabstandet und berührungslos zum optischen Teil 2 angeordnet. Im Ausführungsbeispiel ist in vorteilhafter Weise ein entsprechend ausgebildetes weiteres Koppelelement 14 an dem zweiten Haptikbügel 7 ausgebildet. Dieses ist zum Koppeln mit einem weiteren Gegenkoppelelement 15, welches im optischen Teil 2 ausgebildet ist, ausgebildet. Das in Fig. 2 gezeigte Ausführungsbeispiel unterscheidet sich geringfügig von der Intraokularlinse 1 gemäß Fig. 1a. Es ist in Fig. 1a ein zusätzlich umlaufender Anschluss 2a vorgesehen. Dieser hier umlaufende Anschluss 2a umgibt den optischen Teil 2 und weist keine optische Abbildefunktion auf. Dieser umlaufende Anschluss 2a umgibt den optischen Teil 2 in Umlaufrichtung um die optische Hauptachse A zumindest bereichsweise. Der Anschluss 2a erstreckt sich insbesondere über zumindest ein Segmentstück beziehungsweise ein Ringsegment, welches im Bereich der Haptik 3 an dem Äquator des optischen Teils 2 angeordnet ist. Bei dem Beispiel in Fig. 1a ist im Unterschied zu Fig. 2 das Gegenkoppelelement 9 in diesem Anschluss 2a ausgebildet. Entsprechend ist auch das Gegenkoppelelement 15 in diesem Anschluss 2a ausgebildet. Der Anschluss 2a kann als Ring oder als Segmentstück ausgebildet sein.

Das Gegenkoppelelement 9, 15 kann in seiner Lochgeometrie eckenfrei, beispielsweise rund oder oval sein. Es kann in seiner Lochgeometrie auch eckig sein, beispielsweise viereckig. Das Koppelelement 8, 14, insbesondere der Koppelsteg 13, kann in seiner Geometrie eckenfrei oder eckig sein. Insbesondere ist die Geometrie der Koppelelemente 8, 14 an die Geometrie der Gegenkoppelelemente 9, 15 angepasst. Dadurch ist ein einfaches und genaues Ineinanderführen ermöglicht.

In Fig. 3 ist in einer Schnittdarstellung entlang der Schnittlinie III-III in Fig. 2 der ungekoppelte Zustand der Intraokularlinse 1 bezüglich des Koppelelements 8 mit dem Gegenkoppelelement 9 dargestellt. Es ist zu erkennen, dass der Koppelsteg 13 den Verbindungsbalken 11 axial überragt. Die L-Form des Koppelelements 8 ist in Fig. 3 gezeigt.

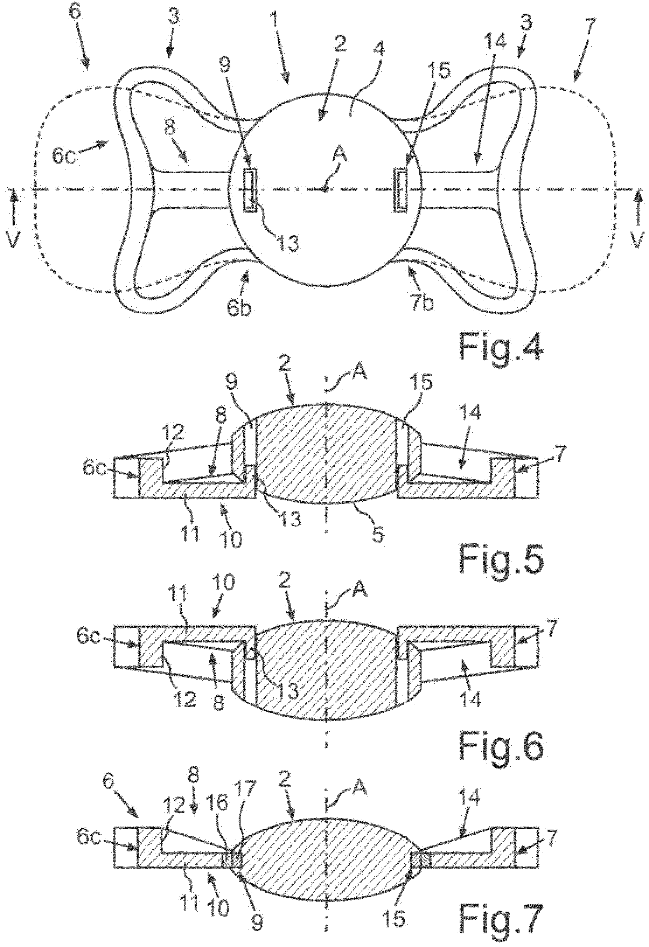

In Fig. 4 ist die Intraokularlinse 1 gemäß Fig. 2 und Fig. 3 in einer Draufsicht gezeigt. Allerdings ist hier der gekoppelte Zustand zwischen dem Koppelelement 8 und dem Gegenkoppelelement 9 gezeigt. Ebenso ist hier der gekoppelte Zustand zwischen dem Koppelelement 14 und dem Gegenkoppelelement 15 gezeigt.

Zum Erreichen dieses gekoppelten Zustands kann ausgehend von der Darstellung in Fig. 2 und 3 mit einem nicht gezeigten Hilfswerkzeug das Koppelelement 8 zum Gegenkoppelelement 9 hin gezogen werden. Dadurch wird dann auch bewirkt, dass das Koppelelement 8 mit dem Gegenkoppelelement 9 gekoppelt wird.

Das Hilfswerkzeug kann beispielsweise einen Haken aufweisen, mit welchem der Koppelsteg 13 hintergriffen werden kann und zum Gegenkoppelelement 9 hingezogen werden kann. Es kann allgemein betrachtet auch vorgesehen sein, dass das Koppelelement 8 einen Eingriffsbereich 20 aufweist. In diesen kann das Hilfswerkzeug eingreifen, um das Koppelelement 8 zum Gegenkoppelelement 9 zu ziehen. Der Eingriffsbereich 20 kann beispielsweise in dem Verbindungsbalken 11 ausgebildet sein. Er kann ein Sackloch oder ein durchgängiges Loch sein. Der Eingriffsbereich 20 ist insbesondere beabstandet zum Koppelsteg 13 ausgebildet. Einen entsprechenden Eingriffsbereich kann auch das Koppelelement 14 aufweisen.

Wie dazu in der Schnittdarstellung in Fig. 5, die einen Schnitt entlang der Schnittlinie V-V in Fig. 4 zeigt, zu erkennen ist, greift der Koppelsteg 13 in dieses Gegenkoppelelement 9 ein. Es ist daher ein axial überlappendes Ineinandergreifen zwischen dem Gegenkoppelelement 9 und dem Koppelsteg 13 bewirkt. Damit ist auch erreicht, dass ein Außenrandsegment 6c des ersten Haptikbügels 6 in einem radial geringeren Abstand zum optischen Teil 2 angeordnet ist, als im ungekoppelten Zustand gemäß Fig. 2 und Fig. 3. Selbiges kann auch für ein Außenrandsegment 7c des Haptikbügels 7 erfolgen. Dieses Außenrandsegment 6c wird somit zu dem optischen Teil 2 hin gezogen. Durch den gekoppelten Zustand zwischen dem Koppelelement 8 und dem Gegenkoppelelement 9 ist diese zum optischen Teil 2 hin verlagerte Position auch dauerhaft fixiert gehalten. Der Koppelsteg 13 ist in dieses Gegenkoppelelement 9 eingehängt. Die Intraokularlinse 1 weist somit eine integrierte Arretiereinheit auf, die das Koppelelement 8 und das Gegenkoppelelement 9 aufweist. Insbesondere sind auch das Koppelelement 14 und das Gegenkoppelement 15 Bestandteil dieser Arretiereinheit.

In Fig. 6 ist in einer Schnittdarstellung analog zu Fig. 5 eine alternative Ausführung gezeigt. Bei dieser greift der Koppelsteg 13 somit nicht von der zweiten optischen Seite 5 her in das Gegenkoppelelement 9 ein, sondern greift von der ersten optischen Seite 4 in das Gegenkoppelelement 9 ein.

In Fig. 7 ist in einer Schnittdarstellung ein weiteres, nicht von der Erfindung umfasstes Ausführungsbeispiel gezeigt. Im Unterschied zu Fig. 2 bis Fig. 6 ist hier kein Koppelsteg 13 vorgesehen. Vielmehr ist das Verbindungsteil 10 insbesondere mit einem Verbindungsbalken 11 ausgebildet. An dessen Ende, welches dem optischen Teil 2 zugewandt ist, ist ein Magnet 16 angeordnet. Im optischen Teil 2 ist ein Gegenmagnet 17 angeordnet. In Fig. 7 ist der bereits gekoppelte Zustand zwischen den Magneten 16 und 17 gezeigt. Damit ist auch eine Kopplung zwischen dem Koppelelement 8 und dem Gegenkoppelelement 9 gebildet. Entsprechend kann auch die Ausgestaltung eines vorzugsweise vorhandenen weiteren Koppelelements 14 und eines weiteren vorhandenen Gegenkoppelelements 15 sein.

In einem weiteren Ausführungsbeispiel kann vorgesehen sein, dass ein Koppelelement 8 mit einem Koppelsteg 13 vorhanden ist, welches in ein Gegenkoppelelement 9 mechanisch direkt eingreift. Ein weiteres Koppelelement 14 kann vorgesehen sein, welches dann einen derartigen Magneten 16 aufweist. Bei dieser Ausführung kann dann das Gegenkoppelement 15 einen entsprechenden Gegenmagneten 17 aufweisen. Es sind somit bei einer Intraokularlinse 1 dann unterschiedliche Konzepte von Koppelelementen und Gegenkoppelelementen realisiert.

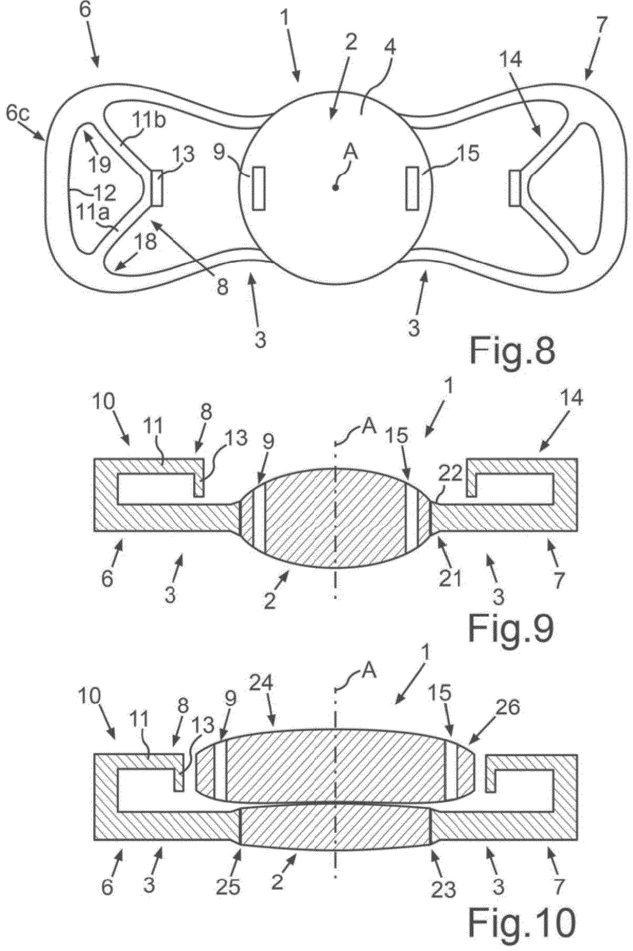

In Fig. 8 ist in einer vereinfachten Draufsicht ein weiteres Ausführungsbeispiel einer Intraokularlinse 1 gezeigt. Bei diesem Ausführungsbeispiel ist im Unterschied zu den bisherigen Beispielen vorgesehen, dass das Koppelelement 8 nicht nur einen Verbindungsbalken 11 aufweist, sondern zwei separate Verbindungsbalken 11a und 11b aufweist. Es können auch mehr als zwei derartige separate Verbindungsbalken realisiert sein. Wie in Fig. 8 zu erkennen ist, mündet der erste Verbindungsbalken 11a an einer ersten Stelle 18 an die Innenseite 12 des ersten Haptikbügels 6. Der zweite Verbindungsbalken 11b mündet an einer dazu unterschiedlichen zweiten Stelle 19 an die Innenseite 12. Insbesondere kann vorgesehen sein, dass die beiden Verbindungsbalken 11a und 11b eine V-Form bilden. Die beiden Enden der Verbindungsbalken 11a und 11b, welche dem optischen Teil 2 zugewandt sind, können miteinander verbunden sein. Sie können einstückig miteinander ausgebildet sein. An diesem zusammengeführten Ende kann der Koppelsteg 13 angeordnet sein. Durch eine derartige Ausgestaltung kann bei einer entsprechenden Kraft, die auf das Koppelelement 8 einwirkt und die zur optischen Hauptachse A hinwirkt, ein Heranziehen eines Außenrandsegmentes 6c des ersten Haptikbügels 6 zum optischen Teil 2 hin bewirkt werden. Dies ist mit höherer Zugkraft möglich als bei den zuvor beschriebenen Ausführungsbeispielen.

In Fig. 9 ist in einer Schnittdarstellung ein weiteres Ausführungsbeispiel einer Intraokularlinse 1 gezeigt. Bei diesem Ausführungsbeispiel sind der optische Teil 2 und die Haptik 3 separat ausgeführt. Das bedeutet, dass sie jeweils für sich eigene Komponenten sind. Die Haptik 3 weist die Haptikbügel 6, 7 auf. Die Haptik 3 weist des Weiteren eine Aufnahme 21 auf. Diese ist im Ausführungsbeispiel ein umlaufender Ring 22, der vollständig geschlossen ist. Der Ring 22 kann auch ein Anschluss 2a sein, wie dies in Fig. 1a gezeigt ist. Der optische Teil 2 ist in dem Ring 22 aufgenommen und gehalten. Die Haptik 3 mit den Haptikbügeln 6, 7 und dem Ring 22 bilden ein Trägerskelett. Die Haptik 3 ist einstückig ausgebildet.

In Fig. 10 ist ein weiteres Ausführungsbeispiel einer Intraokularlinse 1 in einer Schnittdarstellung gezeigt. Die Intraokularlinse 1 weist eine Basislinse 23 auf. Die Basislinse 23 weist einen ersten optischen Teil 2 auf. Die Basislinse 23 weist eine Haptik 3 auf. Die Haptik 3 weist die Haptikbügel 6, 7 auf. Diese können bezüglich der Orientierung entsprechend der Ausgestaltung in Fig. 9 ausgebildet sein. Diese können, ebenso wie die Haptikbügel 6, 7 in dem Ausführungsbeispiel in Fig. 9, auch anderweitig angeordnet und orientiert sein. Beispielsweise so wie in Fig. 3 und 5 oder wie in Fig. 8.

Die Basislinse 23 ist im Ausführungsbeispiel vorzugsweise einstückig ausgebildet. Des Weiteren weist die Intraokularlinse 1 einen zweiten optischen Teil 24 auf. Dieser zweite optische Teil 24 ist separat zur Basislinse 23 ausgebildet. Der zweite optische Teil 24, der auch eine Linse darstellt, ist in Reihe zum ersten optischen Teil 2 angeordnet. Dadurch ist ein Optiksystem 25 der Intraokularlinse 1 gebildet. Insbesondere kann der zweite optische Teil 24 an dem ersten optischen Teil 2 anliegen. Der zweite optische Teil 24 ist mit dem ersten optischen Teil 2 positionsstabil gekoppelt (in Fig. 10 nicht dargestellt). Der zweite optische Teil 24 ist vorzugsweise größer als der erste optische Teil 2 ausgebildet. Dies bedeutet, dass der zweite optische Teil 24 in Richtung senkrecht zur optischen Hauptachse A größer ist, als der erste optische Teil 2. Es ist dadurch ein radialer Überstand 26 des zweiten optischen Teils 24 im Vergleich zum ersten optischen Teil 2 gebildet. In diesem Überstand 26 ist vorzugsweise zumindest ein Gegenkoppelelement 9, 15 ausgebildet.

## Patentansprüche

1. Intraokularlinse (1) mit zumindest einem optischen Teil (2, 24) und mit einer Haptik (3), die mit dem optischen Teil (2, 24) gekoppelt ist, und mit einer optischen Hauptachse (A), die eine Vorderseite (4) und eine Rückseite (5) des optischen Teils (2, 24) durchdringt, wobei die Haptik (3) zumindest einen Haptikbügel (6, 7) aufweist,
wobei
an dem Haptikbügel (6, 7) ein Koppelelement (8, 14) der Intraokularlinse (1) integriert ist, und an dem optischen Teil (2, 24) oder an einem mit dem optischen Teil (2, 24) gekoppelten Anschluss (2a) ein Gegenkoppelelement (9, 15) der Intraokularlinse (1) integriert ist, wobei das Koppelelement (8, 14) zum direkten Koppeln mit dem Gegenkoppelelement (9, 15) ausgebildet ist und wobei im gekoppelten Zustand von Koppelelement (8, 14) und Gegenkoppelelement (9, 15) ein Außenrandsegment (6c, 7c) des Haptikbügels (6, 7) in einem radial geringeren Abstand zum optischen Teil (2) angeordnet ist als im ungekoppelten Zustand von Koppelelement (8, 14) und Gegenkoppelelement (9, 15), wobei der Haptikbügel (6, 7) ein Ende (6a, 6b, 7a, 7b) aufweist, das mit dem optischen Teil (2, 24) oder dem Anschluss (2a) an einer ersten Stelle direkt verbunden ist, wobei das Koppelelement (8, 14) entfernt zu dem Ende (6a, 6b, 7a, ,7b) an dem Haptikbügel (6, 7) angeordnet ist, wobei das Koppelelement (8, 14) ein Verbindungsteil (10) aufweist, mit welchem das Koppelelement (8, 14) mit dem Haptikbügel (6, 7) direkt dauerhaft verbunden ist, und das Koppelelement (8, 14) einen Koppelsteg (13) aufweist, der an dem Verbindungsteil (10) endseitig ausgebildet ist, wobei der Koppelsteg (13) zum direkten Eingriff in das Gegenkoppelelement (9, 15) vorgesehen und das Verbindungsteil (10) im ungekoppelten Zustand frei kragend in Richtung des optischen Teils (2) angeordnet ist und berührungslos zum optischen Teil (2, 24) angeordnet ist,
**dadurch gekennzeichnet, dass**
das Verbindungsteil (10) zumindest einen, insbesondere geradlinigen, Verbindungsbalken (11) aufweist, der von einer Innenseite (12) des Haptikbügels (6, 7) absteht und der sich in radialer Richtung zum optischen Teil (2, 24) hin erstreckt.

2. Intraokularlinse (1) nach Anspruch 1,
**dadurch gekennzeichnet, dass**
der Verbindungsbalken (11) und der Koppelsteg (13) ein im Querschnitt L-förmiges Teil bilden.

3. Intraokularlinse (1) nach einem der vorhergehenden Ansprüche,
**dadurch gekennzeichnet, dass**
der gekoppelte Zustand durch das direkte Ineinandergreifen zwischen dem Koppelelement (8, 14) und dem Gegenkoppelelement (9, 15) selbständig und ohne weitere externe Halteteile gehalten ist.

4. Intraokularlinse (1) nach einem der vorhergehenden Ansprüche,
**dadurch gekennzeichnet, dass**
das Koppelelement (8, 14) ein Verbindungsteil (10) aufweist, mit welchem das Koppelelement (8, 14) mit dem Haptikbügel (6, 7) direkt und dauerhaft verbunden ist, wobei das Verbindungsteil (10) zumindest zwei separate, insbesondere geradlinige, Verbindungsbalken (11a, 11b) aufweist, die an unterschiedlichen Stellen (18, 19) von einer Innenseite (12) des Haptikbügels (6, 7) abstehen und die sich in Richtung des optischen Teils (2) erstrecken, wobei die Verbindungsbalken (11a, 11b) an ihren Enden, die dem optischen Teil (2, 24) zugewandt sind, insbesondere einstückig, miteinander verbunden sind.

5. Intraokularlinse (1) nach einem der vorhergehenden Ansprüche,
**dadurch gekennzeichnet, dass**
das Gegenkoppelelement (9, 15) ein Durchgangsloch ist.

## Claims

1. Intraocular lens (1) having at least one optical part (2, 24) and having a haptic (3) coupled to the optical part (2, 24), and having an optical principal axis (A) that passes through a front side (4) and a back side (5) of the optical part (2, 24), the haptic (3) comprising at least one haptic loop (6, 7),
wherein
a coupling element (8, 14) of the intraocular lens (1) is integrated on the haptic loop (6, 7) and a mating coupling element (9, 15) of the intraocular lens (1) is integrated on the optical part (2, 24) or on a connector (2a) coupled to the optical part (2, 24), the coupling element (8, 14) being formed for direct coupling to the mating coupling element (9, 15) and, in the coupled state of coupling element (8, 14) and mating coupling element (9, 15), an outer edge segment (6c, 7c) of the haptic loop (6, 7) being arranged at a radially shorter distance from the optical part (2) than in the case of the uncoupled state of coupling element (8, 14) and mating coupling element (9, 15), with the haptic loop (6, 7) having an end (6a, 6b, 7a, 7b) directly connected to the optical part (2, 24) or the connector (2a) at a first point, the coupling element (8, 14) being arranged on the haptic loop (6, 7) at a distance from the end (6a, 6b, 7a, 7b), with the coupling element (8, 14) comprising a connecting part (10), by means of which the coupling element (8, 14) is directly permanently connected to the haptic loop (6, 7), and the coupling element (8, 14) having a coupling web (13) formed on the end face of the connecting part (10), the coupling web (13) being provided for direct engagement with the mating coupling element (9, 15) and in the uncoupled state the connecting part (10) being arranged in cantilevered fashion in the direction of the optical part (2) and arranged so as not to be in contact with the optical part (2, 24),
**characterized in that**
the connecting part (10) has at least one, in particular straight, connecting bar (11) which projects from an inner side (12) of the haptic loop (6, 7) and which extends toward the optical part (2, 24) in the radial direction.

2. Intraocular lens (1) according to Claim 1,
**characterized in that**
the connecting bar (11) and the coupling web (13) form a part with an L-shaped cross section.

3. Intraocular lens (1) according to any one of the preceding claims,
**characterized in that**
the coupled state is held independently and without further external holding parts as a result of the direct engagement between the coupling element (8, 14) and the mating coupling element (9, 15).

4. Intraocular lens (1) according to any one of the preceding claims,
**characterized in that**
the coupling element (8, 14) has a connecting part (10), by means of which the coupling element (8, 14) is directly and permanently connected to the haptic loop (6, 7), with the connecting part (10) having at least two separate, in particular straight, connecting bars (11a, 11b) which project from an inner side (12) of the haptic loop (6, 7) at different points (18, 19) and which extend in the direction of the optical part (2), the connecting bars (11a, 11b) being interconnected, in particular in one-piece fashion, at their ends facing the optical part (2, 24).

5. Intraocular lens (1) according to any one of the preceding claims,
**characterized in that** the mating coupling element (9, 15) is a through hole.

## Revendications

1. Lentille intraoculaire (1) avec au moins une partie optique (2, 24) et avec une haptique (3), qui est couplée à la partie optique (2, 24), et avec un axe principal optique (A), qui pénètre dans une face avant (4) et une face arrière (5) de la partie optique (2, 24), l'haptique (3) comportant au moins une pince haptique (6, 7),
un élément de couplage (8, 14) de la lentille intraoculaire (1) étant intégré sur la pince haptique (6, 7), et un contre-élément de couplage (9, 15) de la lentille intraoculaire (1) étant intégré sur la partie optique (2, 24) ou sur un raccord (2a) couplé à la partie optique (2, 24), l'élément de couplage (8, 14) étant formé pour être couplé directement au contre-élément de couplage (9, 15) et un segment de bord extérieur (6c, 7c) de la pince haptique (6, 7) étant disposé, dans l'état couplé de l'élément de couplage (8, 14) et du contre-élément de couplage (9, 15), à une distance radialement plus petite par rapport à la partie optique (2) que dans l'état découplé de l'élément de couplage (8, 14) et du contre-élément de couplage (9, 15), la pince haptique (6, 7) comportant une extrémité (6a, 6b, 7a, 7b) qui est reliée directement à la partie optique (2, 24) ou au raccord (2a) sur un premier emplacement, l'élément de couplage (8, 14) étant disposé de manière éloignée par rapport à l'extrémité (6a, 6b, 7a, 7b) sur la pince haptique (6, 7), l'élément de couplage (8, 14) comportant une partie de liaison (10) à laquelle l'élément de couplage (8, 14) est relié directement durablement à la pince haptique (6, 7), et l'élément de couplage (8, 14) comportant une nervure de couplage (13) qui est formée du côté d'une extrémité sur la partie de liaison (10), la nervure de couplage (13) étant prévue pour venir en prise directement avec le contre-élément de couplage (9, 15) et la partie de liaison (10) étant disposée, dans l'état découplé, librement en porte-à-faux en direction de la partie optique (2) et étant disposée sans contact par rapport à la partie optique (2, 24),
**caractérisée en ce que**
la partie de liaison (10) comporte au moins une poutre de liaison (11), en particulier rectiligne, qui dépasse d'une face intérieure (12) de la pince haptique (6, 7) et qui s'étend dans une direction radiale par rapport à la partie optique (2, 24).

2. Lentille intraoculaire (1) selon la revendication 1,
**caractérisée en ce que**
la poutre de liaison (11) et la nervure de couplage (13) forment une partie en forme de L dans la section transversale.

3. Lentille intraoculaire (1) selon l'une des revendications précédentes,
**caractérisée en ce que**
l'état couplé est maintenu indépendamment et sans autres parties de maintien extérieures par engrènement direct entre l'élément de couplage (8, 14) et le contre-élément de couplage (9, 15).

4. Lentille intraoculaire (1) selon l'une des revendications précédentes,
**caractérisée en ce que**
l'élément de couplage (8, 14) comporte une partie de liaison (10), avec laquelle l'élément de couplage (8, 14) est relié directement et durablement à la pince haptique (6, 7), la partie de liaison (10) comportant au moins deux poutres de liaison (11a, 11b) séparées, en particulier rectilignes, qui dépassent sur différents endroits (18, 19) de la face intérieure (12) de la pince haptique (6, 7) et s'étendent en direction de la partie optique (2), les poutres de liaison (11a, 11b) étant reliées entre elles sur leurs extrémités qui sont tournées vers la partie optique (2, 24), en particulier d'un seul tenant.

5. Lentille intraoculaire (1) selon l'une des revendications précédentes,
**caractérisée en ce que**
le contre-élément de couplage (9, 15) est un trou traversant.
